# EUROPEAN PATENT APPLICATION

(11) **EP 2 269 568 A2**
(43) Date of publication of application: **05.01.2011**
(21) Application number: 10167793.8
(22) Date of filing: 29.06.2010
(51) Int. Cl.: A61K 8/39, A61K 8/81, A61K 8/894, A61Q 1/10, A61K 8/84, A61K 8/898

(54) **Long-wear and water resistant mascara composition enhancing volume and shine**

(30) Priority: 29.06.2009 US 221285 P
(71) Applicant: L'OREAL S.A., 75008 Paris Cedex (FR)
(72) Inventor: Bui, Hy Si, New Jersey, NJ 08854 (US); Kanji, Mohamed, New Jersey, NJ 08837 (US); Li, Chunhua, New Jersey, NJ 07076 (US); Bavouzet, Bruno Thierry, New Jersey, NJ 07030 (US)
(74) Representative: Le Coupanec, Pascale A.M.P.

(57) **Abstract**

The present invention is directed to long wearing, water-resistant, color enhanced eye makeup compositions having a unique texture and contribution to volume comprising an oil-soluble polar modified polymer, a polyamine, a sugar silicone surfactant and an alkyl ethoxylated polymer wax.

## Description

### FIELD OF THE INVENTION

The present invention generally relates to a novel mascara composition and method of making-up eye lashes. More particularly, the present invention relates to a mascara composition having excellent gloss and volumizing properties, as well as long-wear properties.

### BACKGROUND OF THE INVENTION

It has been known that mascaras are usually structured with waxes. The conventional waxes used in mascara formulations have crystals that are large enough to diffract/scatter light, giving the formula an opaque and dull appearance.

It is also well known in the industry that one way of formulating a mascara composition which has long wear properties is to make it either anhydrous or include latex film formers based on oil-in-water emulsions.

Both techniques have numerous drawbacks. First, they are somewhat expensive. Second, they can be difficult to formulate with due to the large solid content load required, making them unstable, or sensitive to added ingredients.

Therefore, it is desirable to provide an eye makeup composition which provides excellent shine, color deposition, and long-wear properties.

### BRIEF SUMMARY OF THE INVENTION

A first aspect of the present invention is directed to long wearing, water-resistant, color enhanced eye makeup compositions having a unique texture and contribution to volume. The compositions include: (a) at least one oil soluble polar modified polymer; (b) at least one sugar silicone surfactant; (c) at least one polyamine; and (d) at least one alkyl ethoxylated polymer wax.

The present invention also relates to long wearing, water-resistant, color enhanced eye makeup compositions, especially mascara compositions, having a unique texture and contribution to volume. The compositions include: (a) a reaction product of at least one polyamine and at least one oil-soluble polar modified polymer;(b) at least one sugar silicone surfactant; and (c) at least one alkyl ethoxylated polymer wax.

The present invention also relates to a mascara composition made by combining:
(a) at least one polyamine;
(b) at least one oil-soluble polar modified polymer;
(c) at least one sugar silicone surfactant; and (d) at least one alkyl ethoxylated polymer wax.

A second aspect of the present invention is directed to a method of making up eyes involving applying the above-disclosed compositions.

The invention relates especially to a method of making-up eyelashes comprising applying onto the eyelashes in an amount sufficient to make-up the eyelashes a composition comprising:
(a) at least one oil-soluble polar modified polymer;
(b) at least one sugar siliconesurfactant;
(c) at least one polyamine; and
(d) at least one alkyl ethoxylated polymer wax.

It has been surprisingly discovered that the above-described compositions yield an eye make-up composition that is water-resistant, long-wearing and provides enhanced volume and shine.

### DETAILED DESCRIPTION OF THE INVENTION

Other than in the operating examples, or where otherwise indicated, all numbers expressing quantities of ingredients and/or reaction conditions are to be understood as being modified in all instances by the term "about".

"Film former" or "film forming agent" or "film forming resin" as used herein means a polymer which, after dissolution in at least one solvent (such as, for example, water and organic solvents), leaves a film on the substrate to which it is applied, for example, once the at least one solvent evaporates, absorbs and/or dissipates on the substrate.

"Keratinous substrates", as used herein, include but are not limited to, skin, hair and nails.

"Substituted" as used herein, means comprising at least one substituent. Non-limiting examples of substituents include atoms, such as oxygen atoms and nitrogen atoms, as well as functional groups, such as hydroxyl groups, ether groups, alkoxy groups, acyloxyalky groups, oxyalkylene groups, polyoxyalkylene groups, carboxylic acid groups, amine groups, acylamino groups, amide groups, halogen containing groups, ester groups, thiol groups, sulphonate groups, thiosulphate groups, siloxane groups, and polysiloxane groups. The substituent(s) may be further substituted.

As defined herein, stability is tested by placing the composition in a controlled environment chamber for 8 weeks at 25°C. In this test, the physical condition of the sample is inspected as it is placed in the chamber. The sample is then inspected again at 24 hours, 3 days, 1 week, 2 weeks, 4 weeks and 8 weeks. At each inspection, the sample is examined for abnormalities in the composition such as phase separation if the composition is in the form of an emulsion, bending or leaning if the composition is in stick form, melting, or syneresis (or sweating). The stability is further tested by repeating the 8-week test at 37°C., 40°C., 45°C., 50°C., and under freeze-thaw conditions. A composition is considered to lack stability if in any of these tests an abnormality that impedes functioning of the composition is observed. The skilled artisan will readily recognize an abnormality that impedes functioning of a composition based on the intended application.

"Volatile", as used herein, means having a flash point of less than about 100°C. "Non-volatile", as used herein, means having a flash point of greater than about 100°C.

As used herein, the expression "at least one" means one or more and thus includes individual components as well as mixtures/combinations.

Other than in the operating examples, or where otherwise indicated, all numbers expressing quantities of ingredients and/or reaction conditions are to be understood as being modified in all instances by the term "about," meaning within 10% to 15% of the indicated number.

"Waterproof" as used herein refers to the ability to repel water and permanence with respect to water. Waterproof properties may be evaluated by any method known in the art for evaluating such properties. For example, a mascara composition may be applied to false eyelashes, which may then be placed in water for a certain amount of time, such as, for example, 20 minutes. Upon expiration of the pre-ascertained amount of time, the false eyelashes may be removed from the water and passed over a material, such as, for example, a sheet of paper. The extent of residue left on the material may then be evaluated and compared with other compositions, such as, for example, commercially available compositions. Similarly, for example, a composition may be applied to skin, and the skin may be submerged in water for a certain amount of time. The amount of composition remaining on the skin after the pre-ascertained amount of time may then be evaluated and compared. For example, a composition may be waterproof if a majority of the product is left on the wearer, e.g., eyelashes, skin, etc. In a preferred embodiment of the present invention, little or no composition is transferred from the wearer.

"Long wear" compositions as used herein, refers to compositions where color remains the same or substantially the same as at the time of application, as viewed by the naked eye, after an extended period of time. Long wear properties may be evaluated by any method known in the art for evaluating such properties. For example, long wear may be evaluated by a test involving the application of a composition to human hair, skin or lips and evaluating the color of the composition after an extended period of time. For example, the color of a composition may be evaluated immediately following application to hair, skin or lips and these characteristics may then be re-evaluated and compared after a certain amount of time. Further, these characteristics may be evaluated with respect to other compositions, such as commercially available compositions.

OIL-SOLUBLE POLAR MODIFIED POLYMER

According to the present invention, compositions comprising at least one oil-soluble polar modified polymer are provided. "Polar modified polymer" as used herein refers to a hydrophobic homopolymer or copolymer which has been modified with hydrophilic unit(s). "Oil-soluble" as used herein means that the polar modified polymer is soluble in oil.

Suitable monomers for the hydrophobic homopolymers and/or copolymers include, but are not limited to, cyclic, linear or branched, substituted or unsubstituted, C2-C20 compounds such as, for example, styrene, ethylene, propylene, isopropylene, butylene, isobutylene, pentene, isopentene, isoprene, hexene, isohexene, decene, isodecene, and octadecene, including all ranges and subranges therebetween. Preferably, the monomers are C2-C8 compounds, more preferably C2-C6 compounds, and most preferably C2-C4 compounds such as ethylene, propylene and butylene.

Suitable hydrophilic unit(s) include, but are not limited to, maleic anhydride, acrylates, alkyl acrylates such as, for example, methyl acrylate, ethyl acrylate, propyl acrylate, and butyl acrylate, and polyvinylpyrrolidone (PVP).

According to the present invention, the polar modified polymer is oil-soluble: that is, the polymer does not contain a sufficient amount of hydrophilic unit(s) to render the entire polymer water-soluble or oil-insoluble. According to preferred embodiments, the polar modified polymer contains the same amount of hydrophobic monomer as hydrophilic unit (1:1 ratio) or more hydrophobic monomer than hydrophilic unit. According to particularly preferred embodiments, the polar modified polymer contains 50% or less hydrophilic unit(s) (based on weight of the polymer), 40% or less hydrophilic unit(s), 30% or less hydrophilic unit(s), 20% or less hydrophilic unit(s), 10% or less hydrophilic unit(s), 5% or less hydrophilic unit(s), 4% or less hydrophilic unit(s), or 3% or less hydrophilic unit(s).

Preferably, the polar modified polymer has from about 0.5% to about 10% hydrophilic units, more preferably from about 1% to about 8% hydrophilic units by weight with respect to the weight of the polymer, including all ranges and subranges therebetween. Particularly preferred hydrophilically modified polymers are ethylene and/or propylene homopolymers and copolymers which have been modified with maleic anhydride units.

According to preferred embodiments of the present invention, the polar modified polymer is a wax. According to particularly preferred embodiments, the polar modified wax is made via metallocene catalysis, and includes polar groups or units as well as a hydrophobic backbone. Suitable modified waxes include those disclosed in U.S. patent application publication no. 20070031361, the entire contents of which is hereby incorporated by reference. Particularly preferred polar modified waxes are C2-C3 polar modified waxes.

In accordance with preferred embodiments of the present invention, the polar modified wax is based upon a homopolymer and/or copolymer wax of hydrophobic monomers and has a weight-average molecular weight Mw of less than or equal to 25 000 g/mol, preferably of 1000 to 22 000 g/mol and particularly preferably of 4000 to 20,000 g/mol, a number-average molecular weight Mn of less than or equal to 15 000 g/mol, preferably of 500 to 12 000 g/mol and particularly preferably of 1000 to 5000 g/mol, a molar mass distribution Mw/Mn in the range from 1.5 to 10, preferably from 1.5 to 5, particularly preferably from 1.5 to 3 and especially preferably from 2 to 2.5, which have been obtained by metallocene catalysis. Also, the polar modified wax preferably has a melting point above 75°C, more preferably above 90°C such as, for example, a melting point between 90°C and 160°C, preferably between 100°C and 150°C, including all ranges and subranges therebetween.

In the case of a copolymer wax, it is preferable to have, based on the total weight of the copolymer backbone, 0.1 to 30% by weight of structural units originating from the one monomer and 70.0 to 99.9% by weight of structural units originating from the other monomer. Such homopolymer and copolymer waxes can be made, for example, by the process described in EP 571 882, the entire contents of which is hereby incorporated by reference, using the metallocene catalysts specified therein. Suitable preparation processes include, for example, suspension polymerization, solution polymerization and gas-phase polymerization of olefins in the presence of metallocene catalysts, with polymerization in the monomers also being possible.

Polar modified waxes can be produced in a known manner from the hompopolymers and copolymers described above by oxidation with oxygen-containing gases, for example air, or by graft reaction with polar monomers, for example maleic acid or acrylic acid or derivatives of these acids. The polar modification of metallocene polyolefin waxes by oxidation with air is described, for example, in EP 0 890 583 A1, and the modification by grafting is described, for example, in U.S. Pat. No. 5,998,547, the entire contents of both of which are hereby incorporated by reference in their entirety.

Acceptable polar modified waxes include, but are not limited to, homopolymers and/or copolymers of ethylene and/or propylene groups which have been modified with hydrophilic units such as, for example, maleic anhydride, acrylate, methacrylate, polyvinylpyrrolidone (PVP), etc. Preferably, the C2-C3 wax has from about 0.5% to about 10% hydrophilic units, more preferably from about 1% to about 8% hydrophilic units by weight with respect to the weight of the wax, including all ranges and subranges therebetween. Particularly preferred hydrophilically modified waxes are ethylene and/or propylene homopolymers and copolymers which have been modified with maleic anhydride units.

Particularly preferred C2-C3 polar modified waxes for use in the present invention are polypropylene and/or polyethylene-maleic anhydride modified waxes ("PEMA," "PPMA." "PEPPMA") commercially available from Clariant under the trade name LICOCARE or LICOCENE, Specific examples of such waxes include products marketed by Clariant under the LicoCare name having designations such as PP207.

Other suitable polar modified polymers include, but are not limited to A-C 573 A (ETHYLENE-MALEIC ANHYDRIDE COPOLYMER; Drop Point, Mettler : 106°C) from Honeywell, A-C 596 A (PROPYLENE-MALEIC ANHYDRIDE COPOLYMER; Drop Point, Mettler : 143°C) from Honeywell, A-C 597 (PROPYLENE-MALEIC ANHYDRIDE COPOLYMER; Drop Point, Mettler : 141°C) from Honeywell, ZeMac® copolymers (from VERTELLUS) which are 1:1 copolymers of ethylene and maleic anhydride, polyisobutylene-maleic anhydride sold under the trade name ISOBAM (from Kuraray), polyisoprene-graft-maleic anhydride sold by Sigma Aldrich, poly(maleic anhydride-octadecene) sold by Chevron Philips Chemcial Co., poly (ethylene-co-butyl acrylate-co-maleic anhydride) sold under the trade name of Lotader (e.g. 2210, 3210, 4210, and 3410 grades) by Arkema, copolymers in which the butyl acrylate is replaced by other alkyl acrylates (including methyl acrylate [grades 3430, 4404, and 4503] and ethyl acrylate [grades 6200, 8200, 3300, TX 8030, 7500, 5500, 4700, and 4720) also sold by Arkema under the Lotader name, and isobutylene maleic anhydride copolymer sold under the name ACO-5013 by ISP.

According to other embodiments of the present invention, the polar modified polymer is not a wax. In accordance with these embodiments of the present invention, the polar modified polymer is based upon a homopolymer and/or copolymer of hydrophobic monomer(s) and has a weight-average molecular weight Mw of less than or equal to 1,000,000 g/mol, preferably of 1000 to 250,000 g/mol and particularly preferably of 5,000 to 50,000 g/mol, including all ranges and subranges therebetween.

In accordance with these embodiments, the polar modified polymer can be of any form typically associated with polymers such as, for example, block copolymer, a grafted copolymer or an alternating copolymer. For example, the polar modified polymer can contain a hydrophobic backbone (such as polypropylene and/or polyethylene) onto which hydrophilic groups (such as maleic anhydride) have been attached by any means including, for example, grafting. The attached groups can have any orienation (for example, atactic, isotactic or syndiotactic along the backbone).

Preferably, the oil soluble polar modified polymer(s) represent from about 1% to about 30% of the total weight of the composition, more preferably from about 2.5% to about 15% of the total weight of the composition, and most preferably from about 5% to about 10%, including all ranges and subranges therebetween.

SUGAR SILICONE SURFACTANT

According to the present invention, compositions comprising at least one sugar silicone surfactant are provided. The sugar silicone surfactant of the present invention has the following formula:

Sach-X-Dn-X-Sach

where Sach represents a saccharide moiety containing multiple hydroxyl groups. Suitable saccharide moieties include, but are not limited to, those based on monosaccharides such as, for example, glucose, fructose, galactose, ribose, mannose, sorbose, etc., and those based one oligosaccharides such as, for example, sucrose, lactose, palatinose, raffinose, lactosucrose, glucosylsucrose, galactosyl-sucrose, xylobiose, etc. Preferably, the saccharide moiety is based on a monosaccharide, most preferably glucose;

X represents a linear or branched, saturated or unsaturated, C1 to C40 hydrocarbon-based group, possibly containing in their chain one or more oxygen, sulphur and/or nitrogen atoms. Preferably, X represents a linear, unsubstituted alkyl group containing at least one N atom, most preferably a linear, unsubstituted alkyl group having 1-6 carbon atoms and at least one N atom;

D represents a silicone based group of the formula R2SiO, where R2 represents a linear or branched, saturated or unsaturated, C1 to C10 hydrocarbon-based group. Preferably, R2 is an unsubstituted C1 to C3 alkyl group (methyl, ethyl, propyl), most preferably a methyl group; and

n represents a number between 1 and 1000, preferably between 100 and 500, more preferably between 250 and 400, and more preferably between 300 and 350, including all ranges and subranges therebetween.

Preferably, such sugar silicone surfactants are prepared by reacting a lactone form of the saccharide with an amino form of the D group, thereby forming an alkyl group X having an N atom between the saccharide moiety and the silicone moiety.

Particularly preferred sugar silicone surfactants include gluconamidoethylaminopropylsilicone, lactobionolactonesiloxane, or a mixture thereof.
According to one embodiment, the sugar silicone surfactant is gluconamidoethylaminopropylsilicone.

Preferably, the sugar silicone surfactant represents from about 0.5% to about 25% of the total weight of the composition, more preferably from about 0.75% to about 15% of the total weight of the composition, and most preferably from about 1% to about 10%, including all ranges and subranges therebetween.

Polyamine Compound

According to the present invention, compositions comprising at least one polyamine compound are provided. In accordance with the present invention, the polyamine compound has at least two primary amine groups available to react with hydrophilic groups of the oil-soluble polar modified polymer.

According to particularly preferred embodiments, the polyamine compound is a polyalkyleneimine, preferably a C2-C5 polyalkyleneamine compound, more preferably a polyethyleneimine or polypropyleneimine. Most preferably, the polyalkylenamine is polyethyleneimine ("PEI"). The polyalkyleneamine compound preferably has an average molecular weight range of from 500-200,000, including all ranges and subranges therebetween.

According to preferred embodiments, compositions of the present invention contain polyethyleneimine compounds in the form of branched polymers. Commercially available examples of such polymers are available from BASF under the tradename LUPASOL or POLYIMIN. Non-limiting examples of such polyethyleneimines include Lupasol^{®} PS, Lupasol^{®} PL, Lupasol^{®} PR8515, Lupasol^{®} G20, Lupasol^{®} G35.

According to other embodiments of the present invention, polyamines such as polyethyleneimines and polypropyleneimines can be in the form of dendrimers. Non-limiting examples of such dendrimers are manufactured by the company DSM, and/or are disclosed in U.S. Pat. No. 5,530,092 and U.S. Pat. No. 5,610,268, the contents of which are hereby incorporated by reference. Commercially available examples of such polymers include polyamidoamine or polypropyleneimine polymers from DENDRITECH sold under the STARBURST® name.

According to other embodiments of the present invention, derivatives of polyalkyleneamines are suitable polyamines. Such derivatives include, but are not limited to, alkylated derivatives, the addition products of alkylcarboxylic acids to polyalkyleneamines, the addition products of ketones and of aldehydes to polyalkyleneamines, the addition products of isocyanates and of isothiocyanates to polyalkyleneamines, the addition products of alkylene oxide or of polyalkylene oxide block polymers to polyalkyleneamines, quaternized derivatives of polyalkyleneamines, the addition products of a silicone to polyalkyleneamines, and copolymers of dicarboxylic acid and polyalkyleneamines. Even further suitable polymamines include, but are not limited to, polyvinylimidazoles (homopolymers or copolymers), polyvinylpyridines (homopolymers or copolymers), compounds comprising vinylimidazole monomers (see, for example, U.S. Pat. No. 5,677,384, hereby incorporated by reference), and polymers based on amino acids containing a basic side chain (preferably selected from proteins and peptides comprising at least 5%, preferably at least 10% of amino acids selected from histidine, lysine and arginine). Such suitable polyamines as described above include those disclosed and described in U.S. patent 6,162,448, the contents of which are hereby incorporated by reference. Commercially available examples of such polymers include polyvinylamine/fonnamide such as those sold under the Lupaminet® name by BASF, chitosan from vegetable origin such as those sold under the Kiosmetine® or Kitozyme® names, or copolymer 845 sold by ISP.

According to preferred embodiments, the at least one polyamine compound is present in the composition of the present invention in an amount ranging from about 0.05 to about 20% by weight, more preferably from about 0.2 to about 10% by weight, more preferably from about 0.5 to about 5% by weight, based on the total weight of the composition, including all ranges and subranges within these ranges.

Preferably, the amount of polyamine compound reacted with the oil-soluble polar modified polymer is such that at least two amine groups on the polyamine compound react with the oil-soluble polar modified polymer to form links or bonds between the amine groups and the hydrophilic groups of the oil-soluble polar modified polymer. The appropriate amount of polyamine compound to react with the oil-soluble polar modified polymer to obtain a reaction product can be easily determined, taking into account the number/amount of reactive amine groups on the polyamine compound and the number/amount of corresponding reactive groups on the oil-soluble polar modified polymer (for example, maleic anhydride groups). According to preferred embodiments, excess oil-soluble polar modified polymer (as determined by the relative number/amount of corresponding reactive groups on the polymer as compared to the reactive amine groups on the polyamine) is reacted with polyamine. Preferably, the polyamine to oil-soluble polar modified ratio is between 0.005 and 1, preferably between 0.006 and 0.5, and preferably between 0.007 and 0.1, including all ranges and subranges therebetween.

ALKYL ETHOXYLATED POLYMER WAXES

The compositions of the present invention comprise at least one alkyl ethoxylated polymer wax that may be selected from di-alkyl, tri-alkyl- and combinations of di-alkyl and tri-alkyl substituted alkyl ethoxylated polymer waxes. Alternatively mono-alkyl, di-alkyl, tri-alkyl, tetra-alkyl and all combinations thereof substituted alkyl ethoxylated polymer waxes. The alkyl group can be saturated or unsaturated, branched or linear and contain a number of carbon atoms from about 12 carbon atoms to about 50 carbon atoms.

The alkyl substitution of the alkyl ethoxylated polymer wax includes mono-alkyl, di-alkyl, tri-alkyl and tetra-alkyl substitution of the polymer wax and combinations thereof. Examples of the polymer waxes that are mono alkyl substituted include: Steareth-100 available as Brij 700 from Uniqema Inc., Pareth alcohols available as Performathox 450, 480 and 490 available from New Phase Technologies, Inc. The di-alkyl substituted polymer waxes include PEG 120 methyl glucose dioleate available as Glutamate DOE-120 and Glucamate DOE-120 both from Chemron Corporation. The tri-alkyl substituted polymer waxes include PEG 120 methyl glucose trioleate available as Glucamate LT from Chemron Corporation. The tetra-alkyl substituted polymer waxes include PEG 150 pentaerythrityl tetrastearate available as Crothix from Croda Corporation.

In the present invention, preferred alky ethoxylated polymer waxes include ethoxylated C₂₀₋₅₀ fatty alcohols having an average molecular weight of the alcohol chain of from about 450 to 550 and an average degree of ethoxylation of from about 2.5 to 95. These alkyl ethoxylated waxes have a melting point ranging from 70 to 100 C. The most preferred waxes are Pareth-10 alcohol which is a mixture of C₂₀₋₄₀ fatty alcohols having an average molecular weight of about 450 and average degree of ethoxylation of about 10, commercially available as Performathox 450, and Pareth-40 alcohol, which is a mixture of C₂₀₋₄₀ fatty alcohols having an average molecular weight of about 450 and an average degree of ethoxylation of about 42, commercially available as Performathox 480, both from New Phase Technologies, Inc.

Preferably, the alkyl ethoxylated polymer wax(es) represent from about 3% to about 30% by weight of the total weight of the composition, more preferably from about 4% to about 20% by weight of the total weight of the composition, and most preferably from about 5% to about 10% by weight of the total composition, including all ranges and subranges therebetween.

Reaction Product

According to preferred embodiments of the present invention, the oil-soluble polar modified polymer is reacted with the polyamine compound, in the presence of water in, at minimum, an amount sufficient to solubilize the polyamine, to form a reaction product. In accordance with the preferred embodiments, the reaction product is water-insoluble.

Although not wanting to be bound by any particular theory, it is believed that at a temperature below 100°C, the reaction of the oil-soluble polar modified polymer with the primary amine group of the polyamine opens the anhydride ring to form a half acid and half amide crosslinked product. However, at a temperature above 100°C, the reaction of the oil-soluble polar modified polymer with the primary amine group of the polyamine opens the anhydride ring to form an imide crosslinked product. The former product is preferred over the latter product. It is not necessary for all amine groups and all hydrophilic groups to react with each other to form the reaction product. Rather, it is possible that the composition may contain free polyamine and/or free oil-soluble polar modified polymer in addition to the reaction product.

Although not wanting to be bound by any particular theory, it is also believed that the polyamine(s) can be non-covalently assembled with the polar modified polymer(s) by electrostatic interaction between an amine group of the polyamine and a hydrophilic group (for example, carboxylic acid group associated with maleic anhydride groups) of the polar modified polymer to form a supramolecule. For example, with specific reference to maleic anhydride groups, in the presence of water these groups can open to form dicarboxylic acid groups which can interact with protonated primary amines of the polyamine through ionic interaction to form a polymer-polymer complex with hydrophilic core crosslinkers and a hydrophobic network that act as supramolecular capsule. If a large amount of maleic anhydride groups are present, the secondary amine groups of polyamine are also protonated and interact with alkyl carboxylates.

According to preferred embodiments, the oil-soluble polar modified polymer is in an oil carrier, and the polyamine compound is in an aqueous carrier, and the reaction occurs by combining the oil carrier and the aqueous carrier. Because the oil-soluble polar modified polymer is typically solid at room temperature, the oil carrier is preferably heated to liquefy the polymer prior to combination with the aqueous carrier. Preferably, the oil carrier is heated beyond the melting point of the oil-soluble polar modified polymer, typically up to about 80°C, 90°C or 100°C.

Without intending to be bound by any particular theory, it is believed that the reason for this is that due to the chemical and physical reactions which take place when the oil-soluble polar modified polymer is combined with the polyamine, the subsequent reaction product that is formed is surprisingly and unexpectedly able to entrap large amounts of water molecules within its hydrophobic matrix. The resultant product is eminently capable of forming a film, is self-emulsifying, waterproof. Moreover, the product is both stable and capable of carrying various types of ingredients.

According to other preferred embodiments of the present invention, the oil-soluble polar modified polymer is reacted with the alkyl ethoxylated polymer wax, in the presence of oil to form a reaction product. If the reaction is conducted at a relatively high temperature (for example, above 140°C) and for a long period of time (>5 hours), a signficant amount of the hydrophilic group (for example, carboxylic acid group associated with maleic anhydride groups) of the oil soluble polar modifed polymer reacts with hydroxyl group(s) of the alkyl ethoxylated wax to yield a significant amount of the reaction product. If, however, the reaction is conducted at a relatively low temperature (for example, below 100°C) and for a short period of time (<1 hour), only a small portion of the hydrophilic group of the polar modified polymer reacts with hydroxyl group(s)of the alkyl ethoxylated polymer wax to yield a minor amount of reaction product. Depending upon desired application, a minor amount or a significant amount of the reaction product may be desired.

OPTIONAL INGREDIENTS

According to preferred embodiments, the oil-soluble polar modified polymer is in an oil carrier, and the sugar silicone surfactant can be in an oil phase or water phase. Because the oil-soluble polar modified polymer is typically solid at room temperature, the oil carrier is preferably heated to liquefy the wax prior to combination with the aqueous carrier (phase). Preferably, the oil carrier is heated beyond the melting point of the polar modified polymer, typically up to about 80°C, 90°C or 100°C.

WATER

The composition of the present invention can also contain water. The water is typically present in an amount of from about 5% to about 50% by weight, such as from about 10% to about 40% by weight, such as from about 25% to about 35% by weight, including all ranges and subranges therebetween, all weights being based on the total weight of the composition. According to particularly preferred embodiments, sufficient water is present to form a water-in-oil emulsion.

NON-VOLATILE OIL FOR OIL-SOLUBLE POLAR MODIFIED POLYMER

The cosmetic composition of the present invention can comprise at least one non-volatile oil capable of dissolving the oil-soluble polar modified polymer. As used herein, the term "non-volatile" means having a boiling point of greater than about 100 degrees C.

Examples of non-volatile oils that may be used in the present invention include, but are not limited to, polar oils such as:

- hydrocarbon-based plant oils with a high triglyceride content consisting of fatty acid esters of glycerol, the fatty acids of which may have varied chain lengths, these chains possibly being linear or branched, and saturated or unsaturated; these oils are especially wheat germ oil, corn oil, sunflower oil, karite butter, castor oil, sweet almond oil, macadamia oil, apricot oil, soybean oil, rapeseed oil, cottonseed oil, alfalfa oil, poppy oil, pumpkin oil, sesame seed oil, marrow oil, avocado oil, hazelnut oil, grape seed oil, blackcurrant seed oil, evening primrose oil, millet oil, barley oil, quinoa oil, olive oil, rye oil, safflower oil, candlenut oil, passion flower oil or musk rose oil; or caprylic/capric acid triglycerides, for instance those sold by the company Stearineries Dubois or those sold under the names Miglyol 810, 812 and 818 by the company Dynamit Nobel;

- synthetic oils or esters of formula R₅COOR₆ in which R₅ represents a linear or branched higher fatty acid residue containing from 1 to 40 carbon atoms, including from 7 to 19 carbon atoms, and R₆ represents a branched hydrocarbon-based chain containing from 1 to 40 carbon atoms, including from 3 to 20 carbon atoms, with R₆ + R₇ ≥ 10, such as, for example, Purcellin oil (cetostearyl octanoate), isononyl isononanoate, C₁₂ to C₁₅ alkyl benzoate, isopropyl myristate, 2-ethylhexyl palmitate, and octanoates, decanoates or ricinoleates of alcohols or of polyalcohols; hydroxylated esters, for instance isostearyl lactate or diisostearyl malate; and pentaerythritol esters;

- synthetic ethers containing from 10 to 40 carbon atoms;

- C₈ to C₂₆ fatty alcohols, for instance oleyl alcohol; and

- mixtures thereof.

The at least one non-volatile oil, if present, is preferably present in the cosmetic composition of the invention in an amount of from about 0.5% to about 15% by weight, such as from about 1% to about 10% by weight, such as from about 2% to about 5% by weight, all weights based on the total weight of the composition.

VOLATILE SOLVENT

The cosmetic composition of the present invention can comprise at least one volatile solvent. In an embodiment of the present invention, at least one volatile solvent may be present and be chosen from a volatile silicone oil or a volatile non-silicone oil.

Suitable volatile silicone oils include, but are not limited to, linear or cyclic silicone oils having a viscosity at room temperature less than or equal to 6cSt and having from 2 to 7 silicon atoms, these silicones being optionally substituted with alkyl or alkoxy groups of 1 to 10 carbon atoms. Specific oils that may be used in the invention include octamethyltetrasiloxane, decamethylcyclopentasiloxane, dodecamethylcyclohexasiloxane, heptamethyloctyltrisiloxane, hexamethyldisiloxane, decamethyltetrasiloxane, dodecamethylpentasiloxane and their mixtures. Other volatile oils which may be used include KF 96A of 6 cSt viscosity, a commercial product from Shin Etsu having a flash point of 94°C. Preferably, the volatile silicone oils have a flash point of at least 40°C.

Non-limiting examples of volatile silicone oils are listed in Table 1 below.

**Table 1**

| Compound | Flash Point (°C) | Viscosity (cSt) |
|---|---|---|
| Octyltrimethicone | 93 | 1.2 |
| Hexyltrimethicone | 79 | 1.2 |
| Decamethylcyclopentasiloxane (cyclopentasiloxane or D5) | 72 | 4.2 |
| Octamethylcyclotetrasiloxane (cyclotetradimethylsiloxane or D4) | 55 | 2.5 |
| Dodecamethylcyclohexasiloxane (D6) | 93 | 7 |
| Decamethyltetrasiloxane(L4) | 63 | 1.7 |
| KF-96 A from Shin Etsu | 94 | 6 |
| PDMS (polydimethylsiloxane) DC 200 (1.5cSt) from Dow Coming | 56 | 1.5 |
| PDMS DC 200 (2cSt) from Dow Corning | 87 | 2 |
| | | |
| PDMS DC 200 (3St) from Dow Coming | 102 | 3 |

Suitable volatile non-silicone oils may be selected from volatile hydrocarbon oils, alcohols, volatile esters and volatile ethers. Examples of such volatile non-silicone oils include, but are not limited to, volatile hydrocarbon oils having from 8 to 16 carbon atoms and their mixtures and in particular branched C₈ to C₁₆ alkanes such as C₈ to C₁₆ isoalkanes (also known as isoparaffins), isododecane, isodecane, and for example, the oils sold under the trade names of Isopar or Permethyl, the C₈ to C₁₆ branched esters such as isohexyl or isodecyl neopentanoate and their mixtures. Preferably, the volatile non-silicone oils have a flash point of at least 40°C.

Non-limiting examples of volatile non-silicone oils are listed in Table 2 below.

| Table 2 | |
|---|---|
| Compound | Flash Point (°C) |
| Isododecane | 43 |
| Propylene glycol n-butyl ether | 60 |
| Ethyl 3-ethoxypropionate | 58 |
| Propylene glycol methylether acetate | 46 |
| Isopar L (isoparaffin C11-C13) | 62 |
| Isopar H (isoparaffin C11-C12) | 56 |

In general, the at least one volatile solvent, if present, is preferably present in the composition in an amount of from about 5% to about 80% by weight, such as from about 10% to about 60% by weight, and from about 20% to about 40% by weight, all weights based on the total weight of the composition.

The composition of the present invention may also include any one, or more, optional ingredients. Examples thereof include, but are not limited to, colorants such as pigments and dyestuffs, co-solvents, waxes, plasticizers, preservatives, fillers, active ingredients, film formers and sunscreens.
According to one embodiment, the composition may further comprise at least one colorant.

It has surprisingly been discovered that the composition of the present invention forms a long wearing, water-resistant eye makeup composition having a unique texture and feel and enhanced shine. Without intending to be bound by theory, it is believed that the combination of oil-soluble polar modified polymer, alkyl ethoxylated polymer, polyamine and sugar silicone forms a product that is able to entrap large amounts of water molecules within its hydrophobic matrix. Consequently, the composition is water-resistant and long-wear without the need for having to employ conventional film forming polymers. Moreover, the product provides both enhanced shine and volume.

### EXAMPLE

A cosmetic composition was prepared containing the below-disclosed ingredients.

| Phase | Component | Example |
|---|---|---|
| A | C20-C40 Pareth-10 | 7.00 |
| A | Propylene-ethylene-Maleic Anhydride Copolymer | 7.00 |
| A | Isohexadecane | 2.33 |
| A | Iron Oxides | 8.0 |
| A | Isododecane | 30.92 |
| A | Propylparaben | 0.2 |
| B | DI Water | 19.00 |
| B | Disodium EDTA | 0.1 |
| B | Potassium Cetyl Phosphate | 2.00 |
| B | Methylparaben | 0.25 |
| B | Gluconamidoethylaminopropylsilocone (and) Alcohol | 20.00 |
| B | Polyethyleneimine (PEI-35) | 2.00 |
| C | Simethicone | 0.1 |
| D | Phenoxyethanol(and) Methylparaben(and) Isopropylparaben(and) Isobutylparaben(and) Butylparaben | 1.1 |
| | Total | 100 |

### Procedure:

1. In the main beaker A, the following were added:Isododecane, C20-C40 Pareth-10, Polypropylene-ethylene-Maleic Anhydride Copolymer wax, and propylparaben. The contents were then heated to 90°C until all solids melted.
2. Added Iron Oxides into main beaker and started homogenizing batch for 1h at 850 RPM. (Temperature maintained at 85-90°C)
3. In another beaker B, added deionized water, Disodium EDTA, Potassium Cetyl Phosphate, Methylparaben, Gluconamidoethylaminopropylsilocone (and) Alcohol and Polyethyleneimine (PEI-35). Mixed until uniform. Heated contents to 90°C.
4. Slowly added contents of beaker B to beaker A. Then added Simethicone to the mixture. The gel formation was observed in 5 minutes after mixing A and B.
5. During the gel formation, slowed down the mixing speed from 250RPM to 100RPM to 50RPM.
6. Once the gel network became thick enough, changed to sweep blade. Started cooling using 50 RPM.
7. At 35°C, added a mixture of Phenoxyethanol (and) Methylparaben(and) Isopropylparaben (and) Isobutylparaben (and) Butylparaben.
8. Continued cooling to 25°C.

## Claims

1. A mascara composition comprising:
(a) at least one oil-soluble polar modified polymer;
(b) at least one sugar silicone surfactant;
(c) at least one polyamine; and
(d) at least one alkyl ethoxylated polymer wax.

2. The composition according to claim 1, wherein the oil-soluble polar modified polymer is present in an amount of from 1% to 30% by weight, more preferably from 2.5% to 15%, and most preferably from 5% to 10%, based on the weight of the composition.

3. The composition according to anyone of the preceding claims, wherein the oil-soluble polar modified polymer is a polypropylene and/or polyethylene-maleic anhydride modified wax.

4. The composition according to anyone of the preceding claims, wherein the sugar silicone surfactant is gluconamidoethylaminopropylsilicone.

5. The composition according to anyone of the preceding claims, wherein the sugar silicone surfactant is present in an amount of from 0.5% to 25% by weight, more preferably from 0.75% to 15%, and most preferably from 1% to 10%, based on the weight of the composition.

6. The composition according to anyone of the preceding claims, wherein the polyamine is a branched polyethyleneimine.

7. The composition according to anyone of the preceding claims, wherein the polyamine is present in an amount of from 0.05% to 20% by weight, more preferably from 0.2 to 10% by weight, more preferably from 0.5 to 5% by weight, based on the weight of the composition.

8. The composition according to anyone of the preceding claims, wherein the alkyl ethoxylated polymer wax is present in an amount of from 3% to 30% by weight, more preferably from 4% to 20% by weight and most preferably from 5% to 10% by weight based on the weight of the composition.

9. The composition according to anyone of the preceding claims, further comprising water, for example in an amount of from 5% to 50% by weight, such as from 10% to 40% by weight, such as from 25% to 35% by weight, based on the weight of the composition.

10. The composition according to anyone of the preceding claims, further comprising at least one colorant.

11. The composition according to anyone of the preceding claims, wherein the oil-soluble polar modified polymer and the polyamine form a reaction product.

12. The composition according to anyone of the preceding claims, wherein the oil-soluble polar modified polymer and the alkyl ethoxylated polymer wax form a reaction product.

13. The composition according to claim 9, in the form of a water-in-oil emulsion.

14. A method of making-up eyelashes comprising applying onto the eyelashes in an amount sufficient to make-up the eyelashes a composition comprising:
(a) at least one oil-soluble polar modified polymer; (b) at least one sugar silicone surfactant;
(c) at least one polyamine; and
(d) at least one alkyl ethoxylated polymer wax.

15. The method of claim 14, wherein the composition further comprises at least one colorant.
